# EUROPEAN PATENT APPLICATION

(11) **EP 4 620 463 A1**
(43) Date of publication of application: **24.09.2025**
(21) Application number: 24165657.8
(22) Date of filing: 22.03.2024
(51) Int. Cl.: A61K 31/045, A23L 23/00, A61K 8/00, A61K 31/7105, A61K 31/713, A61P 17/00, A61P 17/14

(54) **ACTIVE AGENT FOR USE IN HAIR GROWTH REGULATION**

(71) Applicant: CUTANEON - Skin & Hair Innovations GmbH, 22335 Hamburg (DE)
(72) Inventor: CHÉRET, Jérémy, 13125 Berlin (DE); PAUS, Ralf, 22339 Hamburg (DE); AGRAMUNT MORENO, Julià, 43870 Tarragona (ES)
(74) Representative: WSL Patentanwälte Partnerschaft mbB

(57) **Abstract**

In order to provide a new agent for actively controlling hair growth in subjects, the present invention suggests an active agent for use in hair growth regulation, particularly for use in the treatment of hair growth (stimulation or inhibition), wherein said active agent activates, enhances, inactivates, blocks or dampens the cellular response of the olfactory receptor OR10J1 or interferes with the expression of said receptor. Furthermore, the present invention is directed to compositions for use as a cosmetic or medicament or food supplement in the treatment of hair growth said composition comprising at least one of the aforementioned active agents and at least one auxiliary agent. In addition, a non-therapeutic method of hair growth regulation is disclosed, wherein an effective amount of at least one of the aforementioned active agents is administered to a subject.

## Description

The present invention is directed to an active agent for use in hair growth regulation, particularly for use in the treatment of hair growth, wherein said active agent modulates the activity of an olfactory receptor, including but not limited to activation, enhancement, inactivation, blocking or dampening the cellular response of an olfactory receptor or interfering with the expression of said receptor. Further, the present invention is directed to a composition comprising at least one of the afore-mentioned active agents and to a non-therapeutic method of hair growth regulation, wherein an effective amount of at least one of the afore-mentioned active agents is administered to a subject.

The formation of hair follicles occurs once in the lifetime of healthy mammals, with the number of hair follicles determined in utero. Hair follicle morphogenesis is the generation of the whole hair follicle structure from the epidermis and the mesoderm. During postnatal life the hairfollicle undergoes life-long cyclical transformations where it progresses through stages of rapid growth (anagen), regression (catagen) and relative quiescence (telogen) classified by morphological indicators and molecular markers.

There are a number of common and rare diseases associated with the hair follicle and its growth cycle, such as non-scarring alopecia, scarring alopecia, chronic and acute hair shedding, hypertrichosis or hirsutism. Further, there are a number of other conditions, where the control of unwanted hair on the human body or the promotion of hair growth on areas of the human body is desired, such as drug-induced hair loss (e.g. chemotherapy), radiation-induced hair loss (e.g. radiotherapy), unwanted hair growth due to drugs such as cyclosporine A or diazoxide.

For treating hair growth disorders, the prior art provides e.g. corticosteroids, hormonal modulators and immunosuppressants, all of which being associated with considerable unwanted side effects. The same is true for the piperidinopyrimidine derivative minoxidil and the anthracene derivative dithranol, both having been reported to improve hair growth.

EP 3 766 545 B1 discloses promising active agents for actively controlling hair growth in subjects, wherein said active agent activates, enhances, inactivates, blocks or dampens the cellular response of the taste receptor TAS2R4 or interferes with the expression of said receptor.

In the continuous search for active agents for actively controlling and regulating hair growth in subjects the inventors of the present invention found that the olfactory receptor OR10J1 is involved in regulation and modulation of hair growth.

Particularly, the inventors of the present application have found that an agonist of OR10J1 promotes the anagen stage of hair follicles and increases its elongation. Further, the inventor's findings revealed an increase in OR10J1 expression in the basal CD34+ area in the presence of an OR10J1 receptor agonist.

Concluding, the inventors of the present application have found that the administration of an active agent that activates, enhances, inactivates, blocks or dampens the cellular response of a receptor or interferes with the expression of the olfactory receptor OR10J1 is effective in hair growth regulation.

An "olfactory receptor" describes chemoreceptors typically being expressed in the cell membranes of olfactory receptor neurons and being responsible for the detection of odorants. The olfactory receptors form a multigene family consisting of around 400 genes in humans. The reason for the large number of different olfactory receptors is to provide a system for discriminating between as many different odorants as possible, in order to be able to characterize even "unknown" molecules.

"OR10J1" is the official gene symbol for "Olfactory receptor type 10 subfamily J member 1" and identifies the protein that is encoded by the OR10J1 gene in humans (NCBI Gene ID: 26476; HGNC: OR10J1; NCBI mRNA sequence: NC_000001.11; NCBI protein sequence: AL187856.1).

According to one alternative of the present invention an active agent that activates, enhances, inactivates, blocks or dampens the cellular response of the olfactory receptor OR10J1 is used in the treatment, modulation or regulation of hair growth, wherein said active agent is an agonist or antagonist of the olfactory receptor OR10J1.

An active agent that activates, enhances, inactivates, blocks or dampens the cellular response of a receptor is either an agonist or antagonist/inverse agonist of the receptor, wherein an "agonist" is a substance that binds to a receptor and activates or enhances the receptor to produce the cellular response. An "antagonist" is a substance that binds to a receptor and blocks or dampens the cellular response to an agonist rather than activating or enhancing it like an agonist. An "inverse agonist" is a substance that binds to a receptor and inactivates the cellular response to an agonist rather than activating or enhancing it like an agonist.

Accordingly, an inventive agonist of the olfactory receptor OR10J1 activates or enhances the OR10J1 receptor to produce the cellular response, whereas the inventive antagonist of the olfactory receptor OR10J1 blocks or dampens the OR10J1 receptor response to endogenous agonists, and the inventive inverse agonist of the olfactory receptor OR10J1 inactivates the OR10J1 receptor response to endogenous agonists, to produce the cellular response.

In those embodiments, where the active agent is an agonist of the olfactory receptor OR10J1, said active agent is used in the treatment of unwanted hair growth. In those embodiments, where the active agent is an antagonists/inverse agonists of the olfactory receptor OR10J1, said active agent is used in the treatment of unwanted hair loss.

In specific embodiments of the invention the olfactory receptor agonist/antagonist/inverse agonist used is 2,6-Dimethylheptan-2-ol, a natural odorant with citrus notes, having demonstrated an ability to increase intracellular calcium concentration in Hana3A cells transfected with OR10J1 receptors, therefore classifying this agent as an OR10J1 agonist.

In other specific embodiments of the invention the olfactory receptor agonist/antagonist/inverse agonist used is a OR10J1 agonist selected from trimethyl-cyclopent-3-enyl-butane-1-ol-compounds, wherein the butane-1-ol-residue is saturated or monounsaturated and substituted with one or more methyl residues, such as 2-methyl-4-(2,2,3-trimethyl-cyclopent-3-enyl)-butane-1-ol), 2-ethyl-4-(2,2,3-trimethyl-cyclopent-3-enyl)-but-2-en-1-ol, 3-methyl-5-(2,2,3-trimethyl-cy-clopent-3-enyl)-pent-4-en-2-ol and 3,3-dimethyl-5-(2,2,3-trimethyl-cyclopent-3-enyl)-pent-4-en-2-ol (as disclosed in DE 10 2005 026 801), or from trimethyl-cyclopent-3-enyl-cyclohexenyl-compounds, such as 1-[4-(2,2,3-trimethyl-cyclopent-3-enyl)-cyclohex-3-enyl]-ethanol, 1-[3-(2,2,3-trimethyl-cyclopent-3-enyl)-cyclohex-3-enyl]-ethanol, [4-(2,2,3-trimethyl-cyclopent-3-enyl)-cyclohex3-enyl]-methanol and [3-(2,2,3-trimethyl-cyclopent-3-enyl)-cyclohex-3-enyl]-methanol (as disclosed in US 5,189,013).

Alternatively, the OR10J1 agonist is an aptamer binding to the OR10J1 receptor and activating or enhancing the receptor to produce the cellular response.

The term "aptamer" as used herein refers to DNA, RNA or XNA oligonucleotide or peptide molecules that bind to a specific target molecule, such as receptor molecules.

In other embodiments of the invention the olfactory receptor agonist/antagonist/inverse agonist used is a low molecular OR10J1 antagonist/inverse agonist. Alternatively, the OR10J1 antagonist/inverse agonist is an aptamer binding to the OR10J1 receptor and inactivating, blocking or dampening the receptor to produce the cellular response.

Even though the inventors are not aware of any such instance, it cannot be excluded, that there exist prior art hair growth treatment compositions comprising one of the specific inventive active agents mentioned above as an ingredient, without attributing said ingredient any effect on OR10J1. In this event, such accidentally anticipated active agent may be excluded from the scope of the invention by a corresponding disclaimer. Except for excluding said accidentally anticipated specific active agent said disclaimer shall not further affect the scope of the claims.

Non-limiting examples for active agents being accidentally anticipated in the prior art are
- chemical compounds being attributed no medical, pharmacological or biological activity at all by the corresponding prior art,
- chemical compounds being attributed another medical, pharmacological or biological activity by the corresponding prior art as compared to the herein disclosed specific inventive active agents, and/or
- chemical compounds being attributed the same medical, pharmacological or biological activity by the corresponding prior art as compared to the herein disclosed specific inventive active agents, but not being directed to OR10J1 but being directed to another target (e.g. receptor, enzyme, hormone, metabolite).

According to an alternative of the present invention, an active agent that interferes with the expression of the olfactory receptor OR10J1 is used in hair growth regulation. An active agent that interferes with the expression of the olfactory receptor OR10J1 is either miRNA, siRNA or a ribozyme targeted to the OR10J1 gene or targeted to the mRNA corresponding to the OR10J1 gene.

The term "miRNA" refers to microRNA, i.e. small non-coding RNA molecules containing 18 to 25 nucleotides and functioning in RNA knock-down and post-transcriptional regulation of gene expression. miRNAs function via base-pairing with complementary sequences within mRNA molecules. As a result, these mRNA molecules are knocked-down, by cleavage, destabilization and/or less efficient translation of the mRNA.

The term "siRNA" refers to small interfering RNA, i.e. small non-coding RNA molecules, 18 to 25 base pairs in length. siRNA interferes with the expression of specific genes with complementary nucleotide sequences by degrading mRNA after transcription, thereby preventing translation.

According to the present invention a gene is "targeted" by a miRNA, siRNA or a ribozyme when the miRNA, siRNA or a ribozyme molecule selectively decreases or inhibits the expression of OR10J1. The phrase "selectively decrease or inhibit" as used herein refers to miRNA, siRNA or a ribozyme that affects the expression of OR10J1.

In specific embodiments of the invention miRNA or siRNA interfere with the gene expression of the olfactory receptor OR10J1 by hybridizing under stringent conditions to the gene transcript, i.e. the OR10J1 mRNA, wherein hybridizing "under stringent conditions" means annealing to the target mRNA region, under standard conditions, e.g., high temperature (e.g. < 60 °C for 2 hours) and/or low salt content (e.g. 0.1xSSC) which tend to disfavor hybridization.

According to the present invention one of the above-mentioned active agents is used in the treatment of hair growth. In specific embodiments of the invention the treatment is effected locally, i.e. in, on or at the skin area to be treated. In some embodiments of the invention said treatment is effected topically, wherein the term "topical" refers to a medication that is applied to a particular place on the skin and/or its appendages, such as hair. In specific embodiments the topical application is epicutaneous, meaning that the agonist or antagonist is applied directly to the skin. In other embodiments of the invention the treatment is effected transdermally, wherein the term "transdermal" refers to a medication that is applied across the Stratum corneum into the deeper skin layers, e.g. by injection with a standard needle or microneedles. In other embodiments of the invention the treatment is effected transappendageally, wherein the term "transappendageal" refers to an applied formulation that is permeating the skin via skin appendage structures (such as the hair follicles, sebaceous glands, and sweat glands) into the deeper skin layers. In yet other embodiments of the invention the treatment is effected by oral administration in the form of e.g. capsules or tablets or in the form of food supplement drinks.

The term "treatment" as used herein refers to any action resulting in the change of a physical condition. Particularly, the "treatment of hair growth" refers to any change of an initial hair growth condition, such as unwanted presence of hair, unwanted lack of hair, unwanted slow/fast hair growth, chemotherapy-related hair loss, drug-related hair growth and radiation-related hair loss. In other words, the present invention is directed to any kind of hair growth regulation.

In specific embodiments of the invention the above-mentioned active agent is used as a cosmetic in the treatment of hair growth. Particularly, the use as a cosmetic occurs non-therapeutically, but in order to achieve a change of an initial hair growth condition, such as unwanted presence of hair, unwanted lack of hair, unwanted slow/fast hair growth, wherein said initial condition is not caused by a disease or disorder.

In those embodiments, where the above-mentioned active agent is used as a cosmetic the active agent used should be cosmetically acceptable, wherein "cosmetically acceptable" means that the active agent should not be toxic or harmful or have any other detrimental side effects upon application on hair and/or skin.

In some specific embodiments of the invention the above-mentioned active agent is used as a food supplement for regulating hair growth.

In other specific embodiments of the invention the above-mentioned active agent is used as a medicament in the topical treatment of hair growth disorder, wherein the term "disorder" refers to any functional abnormality or disturbance of the normal healthy condition, and the term "medicament" refers to a substance useful in curing, treating or preventing a condition of disorder.

In some embodiments the above-mentioned active agent is used as a medicament in the topical treatment of a hair growth disorder being selected from the group consisting of nonscarring (non cicatricial) alopecia, such as alopecia areata, telogen effluvium, androgenetic alopecia and anagen effluvium, scarring (cicatricial) alopecia, such as cutaneous lichen planopilaris, discoid lupus erythematosus, dissecting cellulitis and folliculitis decalvans, hypertrichiosis and hirsutism.

In those embodiments, where the above-mentioned active agent is used as a medicament the active agent used should be pharmaceutically acceptable, wherein "pharmaceutically acceptable" means that the active agent should not be toxic or harmful or have any other detrimental side effects upon application on hair and/or skin.

In some embodiments at least one of the inventive active is used as an ingredient of a composition for use as a cosmetic or food supplement or medicament in the treatment of hair growth said composition further comprising at least one auxiliary agent selected from the group consisting of carriers, recipients, adjuvants, diluents, penetration enhancers and disintegrants.

In specific embodiments of such compositions the auxiliary agent is selected from the group consisting of liposomes, nanoparticles, carboxymethyl cellulose, hydroxyethyl cellulose, mineral oil, petrolatum, glycerin, polysorbat 80, hydroxyethyl starch, dextran, and polyethylene glycol.

In the inventive compositions the concentration of the active agent usually is in the range of from 10 to 10.000 µM. In some embodiments the lower limit for the concentration of the active agent is 30 µM or even 100 µM. In some embodiments the upper limit is 3.000 µM or 1.000 µm. This results in preferred ranges of e.g. from 30 to 10.000 µM, from 30 to 3.000 µM, from 10 to 3.000 µM, 100 to 3.000 µM and the like.

Referring to the total weight of an inventive composition the concentration of the active agent may vary in specific embodiments within the range of from 0.001 to 30 wt.-%. In some embodiments the lower limit is 0.01 wt.-%, 0,1 wt.-% or even 1 wt.-%. In some embodiments the upper limit is 20 wt.-%, 10 wt.-% or 5 wt.-%. This results in preferred ranges of e.g. from 0.001 to 10 wt.-%, from 0.01 to 5 wt.-%, from 0.01 to 20 wt.-%, from 1 to 30 wt.-% and the like.

In specific embodiments such compositions further compriseat least one other common active agent being effective in the treatment of hair growth.

In such embodiments the other active agent may for example be selected from hair growth inhibitors, such as an active agent that activates, enhances, inactivates, blocks or dampens the cellular response of the taste receptor TAS2R4 or interferes with the expression of said receptor as disclosed in EP 3 766 545 B1 mentioned above.

In other embodiments the other active agent may be selected from inhibitors of omithine decarboxylase (including Difluoromethylornithine (DFMO)), antiandrogen compounds, inhibitors of 5-α-reductase, inhibitors of androgen receptor, inhibitors of S-adenosyl methionine decarboxylase, inhibitors of γ-glutamyl transpeptidase, inhibitors of adenylosuccinate synthetase, inhibitors of aspartate transcarbamylase, inhibitors of transglutaminase, inhibitors of L-asparagine synthetase, pantothenic acid and its analogues, sulfhydryl reactive compounds, inhibitors of lipoxygenase, inhibitors of cyclooxygenase, inhibitors of nitric oxide synthetase, inhibitors of ornithine amino transferase, inhibitors of cysteine synthesis pathway enzymes, inhibitors of protein kinase C, catechin compounds, green tea polyphenols, non-steroidal angiogen-esis suppressors, inhibitors of arginase, inhibitors of the metabolic pathway for the conversion of glucose to acetyl-CoA, compounds that inhibit the formation of glycoprotein, proteoglycans, and glycosaminoglycans, inhibitors of matrix metalloproteinase, inhibitors of the cholesterol synthesis pathway, inhibitors of DNAtopoisomerase, inhibitors of aminoacyl-tRNA synthetase, inhibitors of the hypusine biosynthesis pathway, compounds that activate androgen conjugation, inhibitors of alkaline phosphatase, inhibitors of protein tyrosine kinase, and compounds that increase cellular ceramide levels. Specific examples include cyproterone acetate, progesterone, acivicin, anthglutin, L-alanosine, guanidino-succinic acid, ethacrynic acid, D-pantothenic acid, pantoyl alcohol, gabaculin, canaline, isonicotinic acid, verapamil, phentolamine, pentosan polysulfate, nafoxidine, tripelennamine, octapine, phloretin, argaric acid, simvastatin, atorvastatin, lovastatin, fluvastatin, mevastatin, N^{G}-methyl-L-arginine, NG-nitro-L-arginine, benzoyl-L-argininamide, L-argininamide, quercetin, apigenin, nordihydroguaratic acid (NDGA), ketoprofen, naproxen, tolmetin, diclofenac, diflunisal, sulindac, thiosalicylic acid, cysteamine, diethyldithiocarbamic acid, D-penicillamine, N-acetyl-L-cysteine, bathocuproine, enalapril, tamoxifen, cimetidine, mycophenolic acid, tetracycline, doxycycline, minocycline, thioridazine, trifluoperizine, 1-(5-isoquinolinylsulfonyl)-2-methylpiperazine, glycyrrhetinic acid, epigallocatechin gallate, epicatechin gallate, epigallocatechin, epicatechin, fusidic acid, and nitroso-acetyl-penicillamine.

In yet other embodiments the other active agent may be selected from preventatives of chemotherapy- or radiation-induced alopecia or hair loss, such as 4-((cyanoimino((1,2,2-trimethylpropyl)amino)methyl)amino)benzonitrile, epidermal growth factor, fibroblast growth factors, including but not limited to keratinocyte growth factor (FGF7), prostaglandins, cyclin dependent kinases, p53 inhibitors, capase-3 inhibitors, N-acyl cysteine, parathyroid hormone antagonist, alpha-tocopherol, cyclosporine, angiotensin receptor blockers, and minoxidil.

Generally, the inventive composition may be used in any formulation suitable for treatment of hair growth. In specific embodiments of the invention the composition is formulated in the form of an ointment, a lotion, a cream, a shampoo, an emulsion, a gel, a spray, a suspension, a capsule, a tablet, a plaster or a sustained release plaster. In other specific embodiments of the invention the composition is formulated in the form of a solution. Said solution may be applied by means of a microneedle device or prior to sonication, electrical stimulation, etc.. Alternatively, the solution may by administered orally such as in the form of e.g. capsules or tablets or in the form of food supplement drinks. A further alternative route of administration is intra-oculary.

As mentioned above, the inventive use of the above-mentioned active agent may also occur non-therapeutically, wherein non-therapeutically refers to a treatment not being directed to curing, treating or preventing a condition of disorder (see above).

Therefore, the present invention is further directed to a non-therapeutic method of hair growth regulation, wherein an effective amount of at least one of the above-mentioned active agent is administered to a subject.

The non-therapeutic method also encloses embodiments, where the above-mentioned active agent is administered to the subject to be treated simultaneously, sequentially or separately together with at least one other active agent useful in the treatment of hair growth (see above).

The following examples show some of the features of specific embodiments of the present invention. However, the skilled reader will understand that those embodiments are just exemplary but do not restrict the inventive idea to exactly the features or the combination of features of the embodiments of the examples.

In the description of the examples it is referred to the following figures, wherein
- Figure 1: shows experimental data according to which there is a significant rise in the number of anagen HFs in groups having been treated with OR10J1 agonist 2,6-Dimethylheptan-2-ol as well as a decrease in catagen HFs,
- Figure 2: shows experimental data according to which there is significant hair shaft elongation in groups having been treated with OR10J1 agonist 2,6-Dimethylheptan-2-ol,
- Figure 3: shows experimental data according to which after 6-days, there is a significant increase in Ki67-positive cells in the hair matrix of the HF in groups having been treated with OR10J1 agonist 2,6-Dimethylheptan-2-ol,
- Figure 4: shows experimental data according to which after 6-days, there is a significant increase in CD34 protein expression in the basal outer root sheath of the HF in groups having been treated with OR10J1 agonist 2,6-Dimethylheptan-2-ol as well as an increase in the number of CD34+ cells,
- Figure 5: shows experimental data according to which in groups having been treated with OR10J1 agonist 2,6-Dimethylheptan-2-ol there is a significant increase in the anagen growth factor IGF-1 and a significant decrease in the catagen induction factor,
- Figure 6: shows experimental data according to which following 6 days of incubation with 2,6-Dimethylheptan-2-ol, the protein expression of OR10J1 significantly in-creases in the basal CD34 outer root sheath region,
- Figure 7: shows experimental data according to which OR10J1 protein expression shows correlation with cell proliferation markers and growth factors,
- Figure 8: shows microscopic hair follicle photographs after MF and Ki67 staining,
- Figure 9: shows experimental data according to which 2,6-Dimethylheptan-2-ol, when in-cubated with siOR10J1, loses the ability to induce anagen, and
- Figure 10: shows experimental data according to which 2,6-Dimethylheptan-2-ol, when in-cubated with siOR10J1, loses the ability to create the hair shaft elongation effect.

### Examples

### 1. Effect of 2,6-Dimethylheptan-2-ol in hair follicle (HF) biology

To test the effect of 2,6-Dimethylheptan-2-ol in hair follicle (HF) biology, HFOC (Hair Follicle Organ Culture) was employed, a whole-organ culture technique, wherein HFs are maintained viable *ex vivo.*

### Regime:

- 3 experimental groups:
   - Vehicle group with just cell culture media
      (WEM, 1%P/S, 0.1% Insulin, 0.02%HC, 0.2%DMSO)
   - 250DT group with 250µM 2,6-Dimethylheptan-2-ol in cell culture media
   - 500DT group with 500µM 2,6-Dimethylheptan-2-ol in cell culture media
- 3 patients, HFs donated while undergoing a hair transplants:
   - 25-30HFs vehicle (10 per patient), 25-30HFs 250DT and 25-30HFs 500DT
   - 7-10 HFs per patient per group: 80-90 HFs in total.

### Immunofluorescence:

Quantitative (immuno-)histomorphometry was employed to assess staining intensity in well-defined reference areas for hair growth in the hair matrix and/or outer root sheath using a panel of antibodies, including Ki67, Cleaved-Caspase3, TGFβ2, CD34, OR10J1, and IGF-1.

Samples were fixed for 10 minutes under various conditions-PFA, acetone, or methanol-followed by blocking or not with serum. Subsequently, samples were incubated overnight with primary antibodies and for 45 minutes at room temperature the next day with secondary antibodies.

### Stats:

Student's t-test in all figures where: *** 0.001, ** 0.01 and * 0.05

### Results:

### a) 2,6-Dimethylheptan-2-ol promotes anagen hair cycle stage and increases HF elongation ex vivo

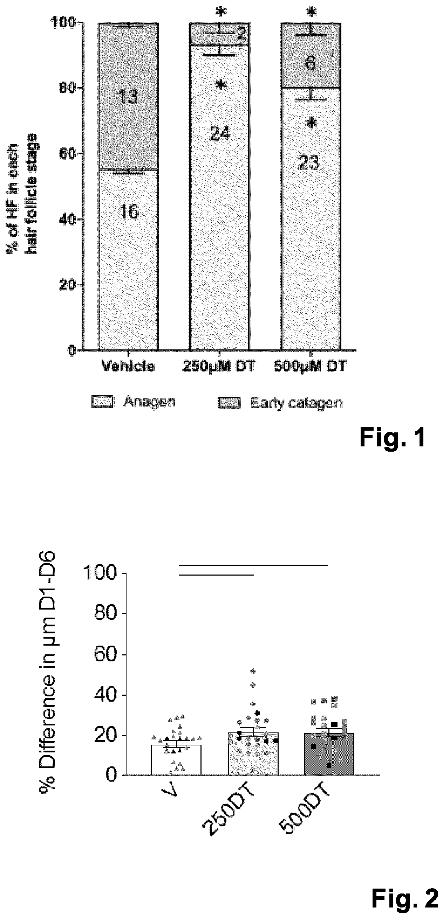

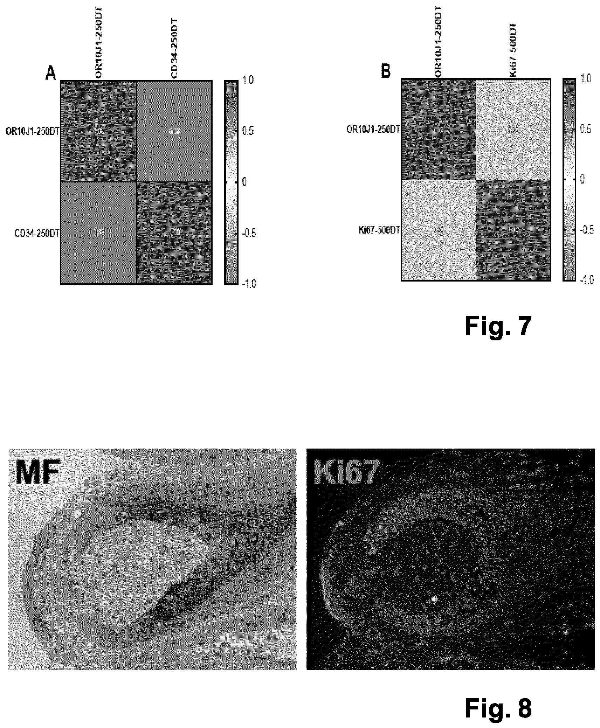

After 6 days in culture, MF and Ki67 staining were performed to evaluate the hair cycle stage. We found a significant rise in the number of anagen HFs in both treated groups as well as a decrease in catagen HFs. The 250DT group induces a greater number if HFs in anagen compared to the 500DT group. For further details see data in attached Figure 1 and HF microscopic photographs of Figure 8.

To assess if the promotion of the anagen stage translates into a physiological response, we measured hair shaft production. Comparing the percentage of hair shaft elongation between day 1 (start of treatment) and day 6 of the culture, we observed that both groups, 250DT and 500DT showed significantly greater hair shaft production compared to the control. For further details see data in attached Figure 2.

### b) 2,6-Dimethylheptan-2-ol increases number of Ki67 proliferative cells in hair matrix keratinocytes and CD34 stem cells in outer root sheath

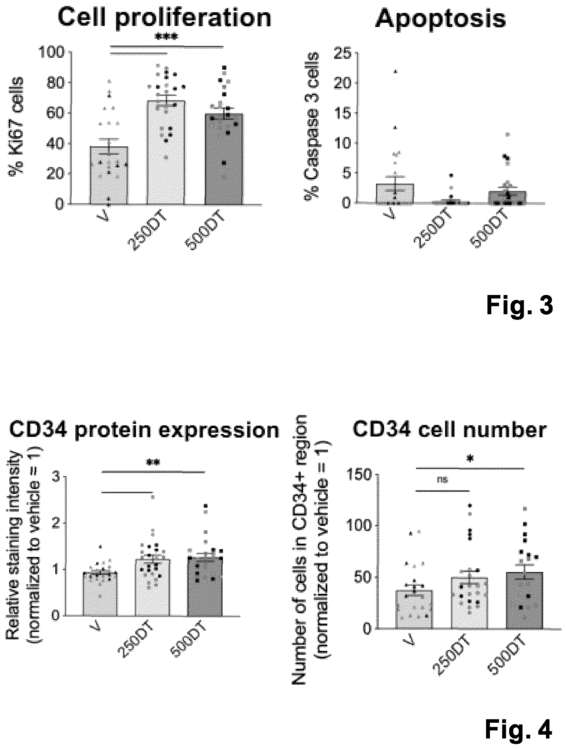

To assess whether macroscopic changes in the HF stages and elongation induced by 2,6-Dimethylheptan-2-ol also translate into molecular changes, we performed qIHM in the hair matrix (HM) and in the outer root sheath (ORS) of the HFs which are well-defined, studied areas where production of mediators cell proliferation/apoptosis for hair growth occurs. Thus, we employed antibodies that target proliferative markers, apoptotic markers, as well as key trophic factors involved in the anagen/catagen transition.
- After 6 days of culture, there is a significant increase in the number of Ki67-positive cells in the HM of the HF treated compared to the control (vehicle) group. Although there is a decrease in the number of apoptotic Caspase 3-positive cells, this difference is not significant. For further details see data in attached Figure 3.
- Next, to assess the level of proliferation in the ORS of the hair follicle, we quantified the amount of protein expression of CD34, a stem cell marker presents in the sub-bulge ORS and implicated in the aetiology of androgenetic alopecia. Interestingly, we observed a significant increase in CD34 protein expression in the sub-bulge ORS after 6 days of 2,6-Dimethylheptan-2-ol treatment in both 250Dt and 500DT groups, along with an increase in the number of CD34+ cells. For further details see data in attached Figure 4.

### c) 2,6-Dimethylheptan-2-ol regulates the levels of TGF-β-2 and IGF-1 protein expression

To investigate whether the rise in anagen HFs is related to the increase of growth factors associated with anagen maintenance (IGF1) and decrease in the ones involved in catagen induction (TGF-β-2), we employed antibodies targeting these specific growth factors.

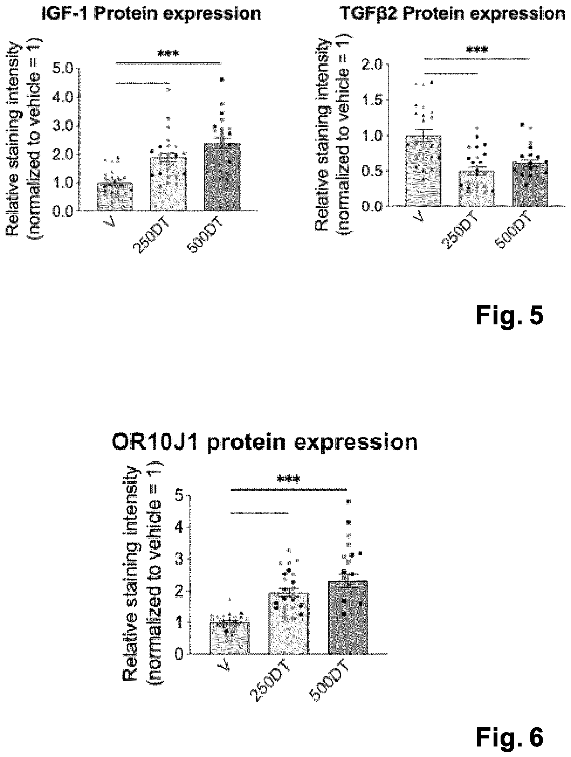

We observed that both groups incubated with DIMETOL (250DT and 500DT) exhibit a significant increase in the anagen growth factor IGF-1 and a significant decrease in the catagen induction factor TGFβ-2, of approximately 2-fold difference in each condition. For further details see data in attached Figure 5.

### d) 2,6-Dimethylheptan-2-ol increases the protein expression of OR10J1

To investigate whether 2,6-Dimethylheptan-2-ol enhances the expression of its receptors, OR10J1, we employed a validated antibody against OR10J1 to quantify the differences in protein expression between the groups incubated with 2,6-Dimethylheptan-2-ol and the vehicle. Following 6 days of incubation with 2,6-Dimethylheptan-2-ol, the protein expression of OR10J1 significantly increases in the sub-bulge CD34+ ORS region compared to the vehicle by approximately 2-fold. For further details see data in attached Figure 6.

### e) OR10J1 protein expression shows correlation with cell proliferation markers and growth factors

After conducting Pearson's correlation coefficient analysis, we identified a significant 0.68 correlation between the protein expression of CD34 and OR10J1 in the 250DT group (Figure 7, A). A parallel correlation analysis with our proliferation marker Ki67 demonstrated a non-significant positive 0.30 correlation between the Ki67-500DT and OR10J1-250DT groups (Figure 7, B).

Next, we evaluated the correlation of CD34 and Ki67 markers with IGF-1 and TGFβ2. We observed a positive correlation between the anagen growth factor IGF-1 and CD34 as well as Ki67 (Figure 7, C-D). Conversely, the catagen induction factor TGFβ2 exhibited a negative correlation with the expression of OR10J1 and the CD34 stem cell marker (Figure 7, E-F).

### 2. Effect of OR10J1-siRNA

Experiments with siRNA targeting the OR10J1 receptor were performed with HFs of a patient as follows.

### Regime:

• 4 experimental groups:
- NTO (control RNA)
- NTO + 250 µM 2,6-Dimethylheptan-2-ol (250 DT)
- siOR10J1 (OR10J1 blocking RNA)
- siOR10J1 + 250 µM 2,6-Dimethylheptan-2-ol (250 DT)
• procedure:

| | |
|---|---|
| - day 0: | isolate, take picture |
| - day 1: | take picture + change medium/add NTO or siRNA |
| - day 2: | rest |
| - day 3: | take picture + change medium/add 2,6-Dimethylheptan-2-ol |
| - day 4: | rest |
| - day 5: | take picture + change medium/add 2,6-Dimethylheptan-2-ol |
| - day 6: | take picture + embed in OCT |

### Results:

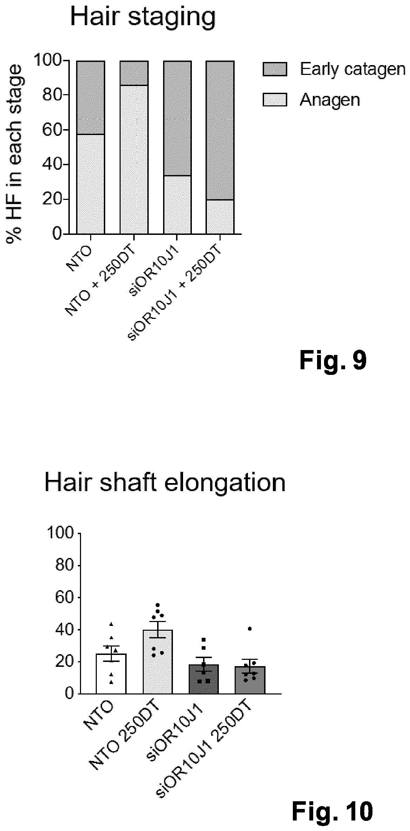

The experiments show that 2,6-Dimethylheptan-2-ol promotes the anagen phase (Figure 9) and hair shaft elongation via the OR10J1 receptors (Figure 10). However, when 2,6-Dimethylheptan-2-ol is incubated with siOR10J1, it loses the ability to induce anagen and to create the hair shaft elongation effect being seen in the control group.

## Claims

1. Active agent for use in the treatment of hair growth, wherein said active agent activates, enhances, inactivates, blocks or dampens the cellular response of the olfactory receptor OR10J1 or interferes with the expression of said receptor.

2. Active agent for use in the treatment of hair growth according to claim 1, wherein the active agent is an agonist of the olfactory receptor OR10J1 for use in the treatment of unwanted hair loss.

3. Active agent for use in the treatment of hair growth according to claim 1, wherein the active agent is an antagonists/inverse agonists of the olfactory receptor OR10J1 for use in the treatment of unwanted hair growth.

4. Active agent for use in the treatment of hair growth according to one of the claims 1 to 3, wherein the active agent is
a) the OR10J1 activating agonist 2,6-Dimethylheptan-2-ol or an aptamer binding to the OR10J1 receptor and activating or enhancing the receptor to produce a cellular response, or
b) an OR10J1 inactivating antagonists/inverse agonists, or an aptamer binding to the OR10J1 receptor and inactivating, blocking or dampening the receptor to produce a cellular response, or
c) an agent acting on a co-receptorto OR10J1 resulting in stimulation or promotion of expression of the receptor OR10J1.

5. Active agent for use in the treatment of hair growth according to claim 1, wherein the active agent is miRNA, siRNA or a ribozyme targeted to OR10J1 or targeted to the mRNA corresponding to the OR10J1 gene.

6. Active agent according to one of the claims 1 to 5 for use as a cosmetic in the treatment of hair growth.

7. Active agent according to one of the claims 1 to 5 for use as a medicament or food supplement in the treatment of hair growth disorder.

8. Active agent for use as a medicament or food supplement according to claim 7, wherein the hair growth disorder to be treated is effluvium, nonscarring alopecia, scarring alopecia, hypertrichosis or hirsutism.

9. Composition for use as a cosmetic or medicament or food supplement in the treatment of hair growth said composition comprising at least one active agent according to one of the claims 1 to 8 and at least one auxiliary agent selected from the group consisting of carriers, recipients, adjuvants, diluents, penetration enhancers, and disintegrants.

10. Composition for use as a cosmetic or medicament or food supplement in the treatment of hair growth according to claim 9, wherein the auxiliary agent is selected from the group consisting of liposomes, nanoparticles, carboxymethyl cellulose, hydroxyethyl cellulose, mineral oil, petrolatum, glycerin, polysorbat 80, hydroxyethyl starch, dextran, and polyethylene glycol.

11. Composition for use as a cosmetic or medicament or food supplement in the treatment of hair growth according to claim 9 or claim 10, further comprising at least one other active agent being effective in the treatment of hair growth.

12. Composition for use as a cosmetic or medicament or food supplement in the treatment of hair growth according to one of the claims 9 to 11, wherein the composition is formulated in the form of an ointment, a lotion, a cream, a shampoo, a gel, a solution, an emulsion, a spray, a suspension, a capsule, a tablet, a plaster or a sustained release plaster.

13. Non-therapeutic method of hair growth regulation, wherein an effective amount of at least one active agent that activates, enhances, inactivates, blocks or dampens the cellular response of the olfactory receptor OR10J1 or interferes with the expression of said receptor is administered to a subject.

14. Non-therapeutic method of hair growth regulation according to claim 13, wherein the active agent is
a) the OR10J1 activating agonist 2,6-Dimethylheptan-2-ol or an aptamer binding to the OR10J1 receptor and activating or enhancing the receptor to produce a cellular response, or
b) an OR10J1 inactivating antagonists/inverse agonists or an aptamer binding to the OR10J1 receptor and inactivating, blocking or dampening the receptor to produce a cellular response, or
c) an agent acting on a co-receptorto OR10J1 resulting in stimulation or promotion of expression of the receptor OR10J1.

15. Non-therapeutic method of hair growth regulation according to one of the claims 13 and 14, wherein the active agent is miRNA, siRNA or a ribozyme targeted to the OR10J1 gene or targeted to the mRNA corresponding to the OR10J1 gene.
